# FASCICULE DE BREVET EUROPEEN

(11) **EP 3 419 589 B1**
(45) Date de publication et mention de la délivrance du brevet: **11.03.2020**
(21) Numéro de dépôt: 17710349.6
(22) Date de dépôt: 23.02.2017
(51) Int. Cl.: A61K 8/73, A61Q 19/06, A61Q 19/08, A61K 8/03

(54) **COMPOSITION COSMÉTIQUE BIPHASE ET SON UTILISATION PAR APPLICATION TOPIQUE**
ZWEIPHASIGE KOSMETISCHE ZUSAMMENSETZUNG UND VERWENDUNG DAVON ZUR TOPISCHEN ANWENDUNG
TWO-PHASE COSMETIC COMPOSITION AND USE THEREOF FOR TOPICAL APPLICATION

(30) Priorité: 25.02.2016 FR 1651554
(43) Date de publication de la demande: 02.01.2019
(73) Titulaire: Pierre Fabre Dermo-Cosmétique, 92100 Boulogne-Billancourt (FR)
(72) Inventeur: DURAN, Violaine, 31860 Labarthe Sur Lèze (FR); CELLIER, Dominique, 31270 Frouzins (FR)
(74) Mandataire: Ipside
(86) Numéro de dépôt international: PCT/FR2017/050399
(87) Numéro de publication internationale: WO 2017/144820

(56) Documents cités:
- EP-A1- 1 424 065
- FR-A1- 2 763 506
- FR-A1- 2 790 758

## Description

La présente invention concerne une composition cosmétique et/ou dermatologique, en particulier destinée à une application topique, du type biphase comportant deux phases non miscibles distinctes pouvant être dispersées l'une dans l'autre par agitation. L'invention concerne également l'utilisation de cette composition dans le domaine cosmétique, notamment en tant qu'amincissant ou pour lutter contre le vieillissement cutané, ainsi qu'un procédé de soin cosmétique mettant en œuvre une telle composition.

On connait des compositions cosmétiques dites biphasés, c'est-à-dire comportant deux phases non miscibles qui sont distinctes l'une de l'autre au repos, et qui sont aptes à former un mélange macroscopiquement homogène lorsque la composition est agitée, l'une des phases se trouvant alors dispersée dans l'autre phase, généralement sous forme de fines gouttelettes ou de microgouttelettes. Typiquement, ces compositions comprennent une phase aqueuse et une phase huileuse. Des exemples de telles compositions sont notamment décrits dans le document EP 0 603 080 ou encore dans le document EP 1 424 065.

De telles compositions comportent de manière classique une association :
- d'un agent émulsionnant, pour permettre la formation d'une émulsion de la phase aqueuse et de la phase huileuse sous agitation, et la stabilisation de cette émulsion, permettant ainsi à un utilisateur de prélever un échantillon homogène des deux phases pour utilisation,
- et d'un agent de déphasage, également nommé agent de démixage, tel qu'un chlorure d'alkyldiméthylbenzylammonium ou un polymère ou copolymère de vinylpyrrolidone, qui assure, après la fin de l'agitation, que les deux phases se séparent et retrouvent rapidement leur état initialement dans lequel elles sont distinctes l'une de l'autre, pour obtenir un aspect visuel plus esthétique et plus attractif pour l'utilisateur.

Pour de telles compositions, le contrôle du temps de déphasage, c'est-à-dire le temps mis par la composition, après agitation, pour revenir à son état initial dans lequel la phase aqueuse et la phase huileuse sont distinctes l'une de l'autre, s'effectue par une réglage précis des proportions relatives de chacun de l'agent émulsionnant et de l'agent de déphasage dans la composition, ces proportions relatives dépendant de l'agent émulsionnant et de l'agent de déphasage particuliers mis en œuvre, ainsi que de l'ensemble des autres constituants contenus dans la composition. Un tel contrôle est contraignant à réaliser.

La présente invention vise à proposer une composition cosmétique et/ou dermatologique de type biphasé, dont le temps de déphasage puisse être contrôlé facilement, en étant à la fois suffisamment long pour que les deux phases restent en dispersion homogène assez longtemps pour permettre une utilisation pratique de la composition par l'utilisateur, et suffisamment court pour retrouver un effet visuel esthétique, en deux phases distinctes, rapidement après l'utilisation.

Des objectifs supplémentaires de l'invention sont que : cette composition soit d'apparence transparente et limpide, tant au repos, lorsque les deux phases sont distinctes, que lors de l'agitation ou après l'agitation, lorsqu'une des deux phases est dispersée dans l'autre ; et qu'au repos, l'interface entre les deux phases soit bien nette.

L'invention vise également à ce que la composition contienne une concentration importante de principe(s) actif(s), pour une efficacité maximale, et que ce(s) principe(s) actif(s) y soient parfaitement solubilisés et stabilisés, de sorte à obtenir l'effet de transparence préférentiellement souhaité.

A l'origine de la présente invention, il a été découvert par les présents inventeurs que, de manière tout à fait surprenante, l'incorporation dans une composition biphase d'un ingrédient de fonction particulière, plus particulièrement l'incorporation dans la phase aqueuse d'un agent d'encapsulation de la phase huileuse, permet de ralentir suffisamment le déphasage de la phase huileuse et de la phase aqueuse pour atteindre l'objectif que s'est fixé la présente invention, c'est-à-dire pour former une composition qui, après agitation, reste sous forme d'une dispersion homogène d'une phase dans l'autre pendant un certain temps, en particulier pendant au moins une trentaine de secondes, puis qui se déphase rapidement, en particulier en moins de quatre minutes, de préférence en moins de deux minutes, pour revenir à son état initial dans lequel les phases sont distinctes l'une de l'autre, et superposées l'une sur l'autre. Un tel résultat avantageux est obtenu par la seule présence dans la phase aqueuse de l'agent d'encapsulation de la phase huileuse, sans qu'il soit besoin de mettre en œuvre une combinaison d'un agent émulsionnant et d'un agent de démixage, et même lorsque la composition est exempte d'alcool, ou contient de l'alcool en quantité inférieure à 15 % en poids, par rapport au poids total de la composition.

Ainsi, il est proposé selon la présente invention une composition cosmétique et/ou dermatologique, destinée notamment à une application par voie topique, du type biphasé, c'est-à-dire qui comprend une phase aqueuse, également nommée phase hydrophile, et une phase huileuse, également nommée phase grasse ou phase lipophile, ces deux phases étant non miscibles, et distinctes l'une de l'autre et superposées l'une sur l'autre au repos, mais aptes à se disperser l'une dans l'autre sous agitation pour former un mélange macroscopiquement homogène. La phase aqueuse contient une quantité comprise entre 0,05 et 0,2 % en poids, par rapport au poids total de la composition, d'un agent d'encapsulation de la phase huileuse choisi parmi les dextrines.

De manière classique en elle-même, on entend au sens de l'invention, par composition biphasé, et par composition comprenant une phase aqueuse et une phase huileuse distinctes l'une de l'autre et superposées l'une sur l'autre au repos, une composition dans laquelle chacune des phases, au repos, est rassemblée en une couche unique qui est entièrement séparée de la couche formée par l'autre phase. Typiquement, les deux phases, au repos, sont séparées par une seule interface, qui est sensiblement plane, et nette. En cela, une composition biphase se distingue totalement d'une émulsion, dans laquelle une phase se trouve dispersée dans l'autre phase de façon stable sous forme d'une multitude de gouttelettes, les interfaces entre les deux phases étant alors multiples.

On entend, par agent d'encapsulation de la phase huileuse, un agent apte à encapsuler des molécules hydrophobes contenues dans la phase huileuse, lorsque les deux phases sont dispersées l'une dans l'autre par agitation, notamment par un effet de formation, par l'agent d'encapsulation, de complexes d'inclusion avec ces molécules hydrophobes, la formation de tels complexes d'inclusion étant temporaire.

La présence d'un tel agent d'encapsulation suffit avantageusement à retarder le déphasage de la phase huileuse et de la phase aqueuse dispersées l'une dans l'autre. Ainsi, après agitation de la composition, l'agent d'encapsulation permet de maintenir la composition dans un état de dispersion homogène de la phase huileuse dans la phase aqueuse pendant un temps suffisamment long pour permettre à un utilisateur de prélever une dose homogène des deux phases sans se presser. L'effet d'encapsulation étant cependant temporaire, il se produit ensuite de manière naturelle, au bout de quelques dizaines de secondes, un déphasage de la composition qui revient alors à son état initial dans lequel les deux phases sont bien distinctes. Une fois initié, ce déphasage est avantageusement particulièrement rapide.

L'effet visuel esthétique retrouvé rapidement après l'utilisation, lorsque la composition est revenue à l'état initial, dans lequel les deux phases sont parfaitement distinctes, est d'autant plus important lorsque, conformément à une caractéristique particulièrement préférée de l'invention, les deux phases sont de couleurs différentes, par exemple lorsque la phase huileuse est incolore, et la phase aqueuse est colorée.

Le temps de retardement du déphasage de la phase huileuse et de la phase aqueuse, c'est-à-dire le moment à partir duquel, après agitation de la composition, la phase aqueuse et la phase huileuse commencent à se séparer l'une de l'autre, et le temps mis par la composition pour revenir à son état initial dans lequel la phase aqueuse et la phase huileuse sont distinctes l'une de l'autre, sont avantageusement contrôlés par la seule concentration de l'agent d'encapsulation dans la composition. Préférentiellement, la concentration en agent d'encapsulation est choisie de telle sorte que le déphasage ne démarre qu'après au moins trente secondes après la fin de l'agitation de la composition, et qu'il soit complet en moins de quatre minutes, de préférence en moins de deux minutes.

L'agent d'encapsulation est contenu dans la composition dans une quantité comprise entre 0,05 et 0,2 %, notamment environ égale à 0,1 %, cette quantité étant exprimée en poids par rapport au poids total de la composition.

L'agent d'encapsulation est choisi parmi les dextrines, de préférence parmi les maltodextrines et les cyclodextrines, et leurs mélanges. Ces molécules sont avantageusement aptes à former des complexes d'inclusion avec les molécules à encapsuler, qui sont alors piégées soit entre des molécules de l'agent d'encapsulation formant une organisation cristalline, soit dans une cavité formée par une structure de la molécule d'agent d'encapsulation.

La ou les dextrines mises en œuvre peuvent être issues de toute origine, notamment d'amidon de blé, de pomme de terre, de maïs, de riz, de tapioca, de légumineuses telles que le pois, le haricot, la fève, la lentille, etc.

L'agent d'encapsulation est de préférence une maltodextrine, par exemple issue d'amidon de maïs, dont les propriétés d'agent gonflant la rendent particulièrement apte à encapsuler les molécules hydrophobes contenues dans la phase huileuse, et à retarder le déphasage de la composition, par renforcement d'interactions colloïdales entre les gouttelettes de la phase huileuse dispersées dans la phase aqueuse.

Les maltodextrines sont des sucres obtenus par hydrolyse acide et/ou enzymatique d'amidon. Elles comprennent un mélange complexe de saccharides linéaires ou ramifiés, et sont typiquement caractérisées par une valeur d'équivalent dextrose (DE) compris entre 1 et 20. Dans la présente description, on englobe cependant, dans le terme maltodextrines, les sirops de glucose, obtenus de manière similaire mais présentant une valeur d'équivalent dextrose (DE) supérieure à 20.

L'agent d'encapsulation peut également être une cyclodextrine. Tout type de cyclodextrine classique en lui-même pour l'homme du métier entre dans le cadre de la présente invention.

La composition selon l'invention répond en outre de préférence à l'une ou plusieurs des caractéristiques suivantes, mises en œuvre séparément ou en chacune de leurs combinaisons techniquement compatibles.

De manière générale, étant destinée à une application par voie topique, en particulier sur la peau, les muqueuses et/ou les cheveux, la composition selon l'invention est telle que l'ensemble de ses constituants sont physiologiquement compatibles.

En outre, les ingrédients contenus dans la composition, en particulier les huiles, sont de préférence choisis pour être adaptés à une application de la composition pour le soin de la peau, des muqueuses et/ou des cheveux, par opposition au démaquillage.

Par ailleurs, les constituants de chacune des phases sont choisis, de même que leurs proportions dans leur phase respective, de telle sorte que chacune des phases soit transparente, chacun des constituants y étant notamment parfaitement solubilisé.

La composition selon l'invention se présente de préférence sous forme liquide. Autrement, elle peut se présenter sous forme de gel, un agent gélifiant étant alors notamment présent dans la phase aqueuse et/ou dans la phase huileuse.

La composition selon l'invention est préférentiellement sensiblement exempte d'agent émulsionnant. On entend, par sensiblement exempte, le fait que la composition ne contient pas d'agent émulsionnant, ou alors en quantité insuffisante pour assurer la création et la stabilisation d'une émulsion à partir de la phase aqueuse et de la phase huileuse. Ainsi, préférentiellement, lorsque la composition selon l'invention contient un émulsionnant, cet émulsionnant y est présent en une quantité inférieure 0,2 %, de préférence inférieure à 0,1 %, et préférentiellement inférieure à 0,05 %, en poids par rapport au poids total de la composition.

La composition selon l'invention est en outre de préférence dépourvue d'agent de démixage / déphasage, tel que du chlorure de benzalkonium ou un polymère ou copolymère de polyvinylpyrrolidone. Elle est de préférence également dépourvue d'alcanes.

La composition selon l'invention est également préférentiellement dépourvue de silice.

Préférentiellement, les constituants de la phase huileuse et les constituants de la phase aqueuse sont choisis de telle sorte que les indices de réfraction de la phase aqueuse et de la phase huileuse soient sensiblement égaux. On entend ici, par sensiblement égaux, le fait que la différence entre l'indice de réfraction d'une des phases et l'indice de réfraction de l'autre des phases est inférieure à 0,01.

Une telle caractéristique assure avantageusement que la composition reste transparente et limpide même après agitation, lorsqu'une des phases est dispersée dans l'autre phase, ce qui lui confère un aspect visuel particulièrement esthétique. Par ailleurs, au repos, il s'ensuit que l'interface entre les deux phases apparait parfaitement nette.

La transparence de la composition peut être vérifiée visuellement, ou par mesure de sa turbidité, de manière classique en elle-même.

Selon une caractéristique particulièrement préférée de l'invention, la phase huileuse contient une huile de silicone, c'est-à-dire une huile comprenant au moins un atome de silicium, ou un mélange de telles huiles. Cette huile est de préférence une huile de silicone non volatile. Les huiles de silicone présentent notamment un effet émollient tout à fait avantageux pour une application par voie topique de la composition selon l'invention, en particulier sur la peau.

L'huile de silicone, ou l'ensemble des huiles de silicone, constituent de préférence le composant majoritaire de la phase huileuse. La quantité totale en huiles de silicone dans la composition est en outre de préférence comprise entre 30 et 60 %, et préférentiellement entre 40 et 50 %, en poids par rapport au poids total de la composition.

L'huile de silicone non volatile est de préférence la diméthicone, qui présente notamment les avantages d'un déphasage rapide après dispersion dans une phase aqueuse, et d'un indice de réfraction relativement bas, plus proche de celui de l'eau que la plupart des autres huiles de silicone.

La phase huileuse peut également contenir une ou plusieurs autres huiles, notamment des huiles hydrocarbonées, en particulier d'origine végétale, telles que des esters d'acides capryliques et d'alcool gras, notamment d'alcools gras en C12 à C18. A titre d'exemple, la phase huileuse peut ainsi contenir une ou plusieurs substances choisies parmi le coco-caprylate et le dicaprylyl carbonate.

La phase huileuse peut contenir tout autre additif liposoluble classique en lui-même pour la formulation de compositions cosmétiques, notamment à usage topique.

Outre de l'eau, la phase aqueuse peut quant à elle contenir un polyol, qui est de préférence choisi parmi la glycérine, l'éthylène glycol, le propylène glycol, le butylène glycol, ou un mélange de tels polyols.

Ce polyol peut autrement consister en un polyéthylène glycol, un polypropylène glycol, tel que le dipropylène glycol, etc.

La mise en œuvre de glycérine est particulièrement préférée dans le cadre de l'invention, en raison notamment de son indice de réfraction relativement élevé (d'une valeur de 1,473), en particulier par rapport à l'eau, et de son effet d'hydratant physiologique. La glycérine présente en particulier une forte capacité de rétention en eau à la surface cutanée, et donc une forte capacité de maintien d'une hydratation de surface.

Le polyol, ou l'ensemble des polyols, constituent de préférence le composant majoritaire de la phase aqueuse. La quantité totale de polyols dans la composition est en outre comprise entre 10 et 20 % en poids, par rapport au poids total de la composition.

Dans des modes de réalisation particuliers de l'invention, la phase aqueuse contient un polyol, ou un mélange de polyols, en tant que composant majoritaire, et la phase huileuse contient une huile de silicone non volatile, ou un mélange de telles huiles, en tant que composant majoritaire.

Préférentiellement, la phase aqueuse contient de la glycérine en tant que composant majoritaire, et la phase huileuse contient de la diméthicone en tant que composant majoritaire. La présence conjointe de ces deux constituants dans la composition selon l'invention s'avère particulièrement avantageuse en termes des propriétés cosmétiques de la composition selon l'invention, en particulier d'efficacité, d'effet sensoriel et d'aspect visuel. En outre, la composition reste transparente tant au repos qu'après agitation.

La phase aqueuse peut en outre contenir un alcool en C1-C6, de préférence non aromatique, tel que l'éthanol, dans une quantité inférieure à 15 % en poids par rapport au poids total de la composition.

Elle peut autrement être exempte de tel alcool.

La phase aqueuse peut également contenir tout autre additif hydrosoluble classique en lui-même pour la formulation de compositions cosmétiques, notamment à usage topique.

Selon une caractéristique préférée de l'invention, la phase huileuse représente 50 à 80 %, de préférence 55 à 80 %, de préférence encore 60 à 70 %, en poids du poids total de la composition. La phase aqueuse représente 20 à 50 %, de préférence 30 à 40 %, en poids par rapport au poids total de la composition.

La composition selon l'invention peut en outre contenir au moins un agent actif par voie topique, ou une pluralité de tels agents actifs.

Ce ou ces agents actifs peuvent être présents aussi bien dans la phase aqueuse, que dans la phase huileuse. Préférentiellement, la composition selon l'invention contient avantageusement au moins un agent actif présent dans la phase aqueuse, et au moins un agent actif présent dans la phase huileuse, pour obtenir une meilleure efficacité pour un effet donné, ou plusieurs effets bénéfiques simultanés.

Pour cela, la forme en deux phases de la composition selon l'invention s'avère tout à fait avantageuse, puisqu'elle permet de réunir dans la même composition des actifs solubles uniquement dans la phase aqueuse, et des actifs solubles uniquement dans la phase huileuse.

La composition selon l'invention peut notamment contenir au moins un agent actif à propriétés amincissantes.

En particulier, en tant que tel agent actif à propriétés amincissantes, la composition selon l'invention peut contenir de la caféine.

De préférence, la quantité de caféine dans la composition est alors supérieure à 0,5 % en poids par rapport au poids total de la composition, de préférence comprise entre 0,8 et 1,2 % en poids par rapport au poids total de la composition, et préférentiellement encore environ égale à 1 % en poids par rapport au poids total de la composition. Une quantité aussi élevée de caféine permet avantageusement de conférer à la composition un effet amincissant particulièrement élevé, en particulier un effet d'atténuation de l'aspect peau d'orange de la peau, plus couramment nommé sous le terme de cellulite et/ou de prévention de l'apparition d'un tel aspect de peau d'orange.

La caféine est de préférence contenue dans la phase aqueuse. A cet effet, lorsque la concentration en caféine de la composition finale visée est supérieure à 0,8 %, la phase aqueuse contient de préférence un alcool en C1-C6, par exemple l'éthanol, qui permet sa complète solubilisation et l'obtention d'une phase aqueuse limpide et transparente.

En outre, toujours afin d'atteindre un objectif de l'invention, qui est que la phase aqueuse soit limpide, et le reste dans le temps, la composition contient avantageusement, de préférence dans la phase aqueuse, une substance stabilisant la caféine.

Les présents inventeurs ont découvert qu'il était possible de stabiliser la caféine à une concentration aussi élevée que 2,5 % en poids dans la phase aqueuse (pour obtenir, après mélange avec la phase huileuse, une concentration finale proche de 1 %), par la présence dans la phase aqueuse de salicylate de triéthanolamine. Une telle substance évite notamment avantageusement les phénomènes de recristallisation de la caféine dans la composition.

A cet égard, la présente invention concerne également, plus généralement, une composition cosmétique et/ou dermatologique de type biphasé, comprenant une phase aqueuse et une phase huileuse distinctes l'une de l'autre, qui contient une quantité supérieure ou égale à 0,8 % en poids, par rapport au poids total de la composition, de caféine, ainsi qu'une substance stabilisante telle que le salicylate de triéthanolamine, et qui présente un aspect limpide. Cette composition peut en outre contenir une huile de silicone non volatile telle que la diméthicone, et/ou un polyol tel que la glycérine, et/ou une petite quantité d'un alcool en C1-C6, tel que l'éthanol, c'est-à-dire une quantité inférieure à 15 % en poids, par rapport au poids total de la composition, par exemple environ égale à 10 % en poids, à plus ou moins 1 % près. Cette composition peut être dépourvue d'agent d'encapsulation de la phase huileuse. Elle peut en outre répondre à l'une ou plusieurs des caractéristiques décrites ci-avant ou ci-après.

La composition selon l'invention peut en outre contenir, en tant qu'agent actif à propriétés amincissantes, de la phloridzine.

La phloridzine est une dihydrochalcone glycosylée appartenant à la famille des flavonoïdes, qui présente notamment un effet antioxydant qui la rend particulièrement avantageuse pour une application dans une composition à visée anti-vieillissement cutané ; ainsi qu'une activité d'inhibition de la différenciation des pré-adipocytes en adipocytes, qui la rendent tout à fait appropriée pour une application cosmétique amincissante. En empêchant que les pré-adipocytes ne se différencient en adipocytes, la phloridzine permet en effet d'éviter l'augmentation d'une surcharge de graisse au niveau du tissu adipeux, et en particulier cellulitique, et limite le stockage des graisses.

La phloridzine peut notamment être contenue dans la composition sous forme pure, ou sensiblement pure. Préférentiellement, elle y est présente sous forme d'un extrait de branches de pommiers (*Pirus malus*), notamment tel que décrit dans le document FR 2 799 121. Un tel extrait se présente de préférence initialement sous forme d'un extrait sec contenant 10 à 25 % en poids de phloridzine, par rapport au poids total de matière sèche, ainsi que 20 à 40 % de polyphénols, dont l'action antioxydante s'avère elle-même avantageusement bénéfique pour les applications cosmétiques. Il peut notamment être obtenu par extraction de branches de pommier broyées par un solvant polaire.

Préférentiellement, la composition selon l'invention contient un mélange de caféine et de phloridzine.

Dans des modes de réalisation particuliers de l'invention, la composition contient au moins un agent anti-vieillissement cutané.

En particulier, cet agent anti-vieillissement peut être extrait de la plante *Argania Spinosa*, couramment nommée arganier. Les extraits de cette plante sont notamment riches en vitamine E et en acides gras essentiels, antioxydants, et participent avantageusement, mis en œuvre dans une composition cosmétique, à la prévention du dessèchement de la peau, ce qui leur confère un effet anti-rides.

Préférentiellement, la phase huileuse de la composition selon l'invention contient de l'huile d'*Argania Spinosa,* et/ou la phase aqueuse contient un extrait hydrophile d'*Argania Spinosa.*

De préférence, la phase huileuse contient de l'huile d'*Argania Spinosa* et la phase aqueuse contient un extrait hydrophile d'*Argania Spinosa,* notamment riche en peptides actifs, pour obtenir une efficacité anti-vieillissement cutané particulièrement importante.

En outre, les présents inventeurs ont maintenant découvert qu'un ester d'alcool gras particulier, le coco-caprylate, permet de solubiliser parfaitement l'huile d'arganier dans une phase huileuse, notamment constituée en majorité d'une huile de silicone, en quantité aussi élevée que 2 % en poids, et même que 10 % en poids, d'huile d'arganier par rapport au poids total de la phase huileuse, si bien que la phase huileuse présente alors un aspect limpide, conformément à l'un des objectifs que s'est fixés la présente invention.

A cet égard, la présente invention concerne également, de manière plus générale, une composition cosmétique et/ou dermatologique de type biphasé, comprenant une phase aqueuse et une phase huileuse distinctes l'une de l'autre, qui contient une quantité supérieure ou égale à 0,1 % en poids, par rapport au poids total de la composition, d'huile d'arganier, ainsi qu'un ester d'acide caprylique et d'alcool gras tel que le coco-caprylate, et qui présente un aspect limpide. Elle peut en outre contenir une huile de silicone non volatile telle que la diméthicone, et/ou un polyol tel que la glycérine. Cette composition peut être dépourvue d'agent d'encapsulation de la phase huileuse. Elle peut en outre répondre à l'une ou plusieurs des caractéristiques décrites ci-avant ou ci-après.

La composition selon l'invention peut en outre comprendre tous autres agents actifs, notamment par voie topique, par exemple choisis parmi les agents hydratants, de préférence physiologiques, les agents émollients, les antioxydants, les bloqueurs de rayonnements ultra violet, les vitamines, les composés auto-bronzants, les huiles essentielles, etc., ou un mélange de tels composés.

Elle peut en outre contenir tout additif classique en lui-même pour une composition cosmétique, par exemple un ou plusieurs des ingrédients ci-après : parfum, colorant, conservateur, agent neutralisant, etc.

Préférentiellement, la composition contient un agent colorant dans une seule des deux phases, de sorte qu'une première des phases, typiquement la phase huileuse, est incolore, et la deuxième des phases, typiquement la phase aqueuse, est colorée. Une telle caractéristique améliore avantageusement l'aspect esthétique de la composition, lorsque celle-ci est au repos, puisque les deux phases apparaissent alors très clairement distinctes l'une de l'autre, avec une interface dont la netteté est alors particulièrement soulignée.

La composition selon l'invention peut en outre contenir un tensioactif non ionique, de préférence siliconé, préférentiellement en petite quantité, inférieure à 0,2 %, notamment inférieure à 0, 1 %, et par exemple inférieure à 0,05 %, en poids par rapport au poids total de la composition. Un tel composant permet avantageusement, lors du retour à l'état initial après agitation, d'éviter le dépôt de gouttelettes de phase huileuse ou de phase aqueuse sur les parois du récipient contenant la composition, en particulier dans la partie de ce récipient vide de composition.

Un tel tensioactif non ionique peut notamment être choisi parmi les alkyl-diméthicone polyols et les alcoxydiméthicone copolyols. A titre d'exemple, il peut ainsi être mis en œuvre dans le cadre de l'invention la substance commercialisée sous la dénomination Abil® WE 09, de dénomination INCI : Cetyl PEG PPG-10/1 Dimethicone / Polyglyceryl-4 isostearate / Hexyl laurate / Pentaerythrityl tetra di-t-butyl hydroxyhydrodrocinnamate.

La composition selon l'invention peut être conditionnée dans tout récipient classique en lui-même, équipé de tout type de moyens de distribution, par exemple conditionnée en flacon pompe ou en spray. Le récipient contenant la composition peut être formé en toute matière, notamment en verre ou en une matière plastique. A titre d'exemple, lorsque la composition contient un agent actif à propriétés amincissantes, tel que la caféine, le récipient contenant la composition peut être formé en matière plastique telle que le polyéthylène. Lorsque la composition contient un agent anti-vieillissement cutané, tel qu'une huile et/ou un extrait d'*Argania Spinosa*, le récipient contenant la composition peut être formé en verre.

Le récipient contenant la composition est en outre du type à compartiment unique, contenant la phase aqueuse et la phase huileuse, pour permettre la dispersion facile de l'une dans l'autre par agitation, extemporanément au moment de l'utilisation.

Selon un autre aspect, la présente invention concerne un procédé de préparation d'une composition selon l'invention, répondant notamment à l'une ou plusieurs des caractéristiques ci-avant. Ce procédé comprend la préparation de chacune de la phase aqueuse et de la phase huileuse indépendamment l'une de l'autre, par solubilisation de chacun des ingrédients dans la phase qui le contient respectivement, puis le mélange de la phase huileuse et de la phase aqueuse ainsi obtenues.

Un autre aspect de l'invention concerne l'utilisation d'une composition selon l'invention, répondant notamment à l'une ou plusieurs des caractéristiques ci-avant, pour le soin cosmétique par application par voie topique sur la peau, les muqueuses et/ou les cheveux, notamment d'un mammifère, en particulier d'un humain.

Selon l'invention, cette utilisation comprend de préférence l'agitation de la composition de sorte à disperser la phase aqueuse et la phase huileuse l'une dans l'autre, et le prélèvement d'une dose de la dispersion ainsi formée pour application par voie topique sur la peau, les muqueuses et/ou les cheveux.

Lorsque la composition selon l'invention contient au moins un agent actif à propriétés amincissantes, elle peut notamment avantageusement être utilisée pour le traitement et/ou la prévention des surcharges adipeuses localisées et/ou de la cellulite, et/ou pour l'obtention d'un effet amincissant, par application par voie topique sur la zone de peau concernée.

Lorsque la composition selon l'invention contient au moins un agent actif anti-vieillissement cutané, elle peut notamment être utilisée pour lutter contre le vieillissement cutané, par application par voie topique sur la zone de peau concernée. La lutte contre le vieillissement cutané peut notamment se traduire par une atténuation ou un retardement des effets du vieillissement de la peau, une restructuration de l'épiderme, un raffermissement de la peau, une atténuation ou une résorption des rides, un renforcement des propriétés protectrices de l'épiderme, et/ou un maintien ou une amélioration de l'hydratation cutanée.

Un autre aspect de l'invention concerne un procédé de soin cosmétique comprenant l'application topique, sur au moins une partie de la peau du corps et/ou du visage d'un mammifère, en particulier d'un humain, d'une composition selon l'invention, répondant notamment à l'une ou plusieurs des caractéristiques ci-avant.

Ce procédé comprend de préférence l'agitation de la composition de sorte à disperser la phase aqueuse et la phase huileuse l'une dans l'autre, et le prélèvement d'une dose de la dispersion ainsi formée pour application par voie topique sur au moins une partie de la peau du corps et/ou du visage.

Ce procédé peut notamment comprendre l'application de la composition, contenant un actif anti-vieillissement cutané, en quantité efficace pour lutter contre le vieillissement cutané, en particulier sur la peau du visage, du cou et le cas échéant des mains.

Il peut comprendre l'application de la composition, contenant un agent actif amincissant, en quantité efficace pour traiter et/ou prévenir les surcharges adipeuses localisées et/ou la cellulite et/ou pour obtenir un effet amincissant, en particulier sur la peau des cuisses, du fessier et du ventre.

Les caractéristiques et avantages de l'invention apparaîtront plus clairement à la lumière des exemples ci-après, fournis à simple titre illustratif et nullement limitatifs de l'invention, avec l'appui des figures 1 à 4, dans lesquelles :
- la figure 1 montre des photographies de flacons contenant, au repos, (a) une composition selon l'invention contenant de la maltodextrine, (b) une composition identique sans maltodextrine ;
- la figure 2 montre des photographies des flacons de la figure 1, immédiatement après agitation, (a) pour la composition selon l'invention contenant de la maltodextrine, (b) pour la composition identique sans maltodextrine ;
- la figure 3 montre des photographies des flacons de la figure 1, 11 secondes après arrêt de l'agitation, (a) pour la composition selon l'invention contenant de la maltodextrine, (b) pour la composition identique sans maltodextrine ;
- et la figure 4 montre des photographies d'un flacon contenant une composition selon l'invention contenant de la cyclodextrine, lors d'un retour au repos après agitation, plus particulièrement a/ 50 secondes, b/ 1 minute et 15 secondes, c/ 2 minutes et 15 secondes, après la fin de l'agitation.

### EXEMPLE 1 - Composition cosmétique amincissante

Il est préparé une composition à effet amincissant comprenant les ingrédients suivants. Pour chacun de ces ingrédients, la concentration est indiquée en pourcentage en poids, par rapport au poids total de la composition.

| Phase aqueuse | (40 %) |
|---|---|
| Glycérine | 10 - 20 % |
| Eau déminéralisée ou eau thermale | qsp 100 % |
| Alcool | 1 - 15 % |
| Caféine | 1 % |
| Acide citrique | 0,005 % |
| Maltodextrine | 0,05 - 0,2 % |
| Extrait de tige de *Pirus malus* | 0,05 - 0,3 % |
| Salicylate de triéthanolamine | 0,1 - 3,0 % |
| Chlorure de sodium | 0,1 - 1,5 % |
| Colorant | qs |

| Phase huileuse | (60 %) |
|---|---|
| Diméthicone | 30 - 59 % |
| Dicaprylyl carbonate | 0 - 20 % |
| Huile de graine de *Camelina sativa* | 0,1 - 4 % |
| Diméthiconol | 0 - 0,1 % |
| Parfum | 0,1 - 1 % |

Les indices de réfraction de la phase aqueuse et de la phase huileuse sont avantageusement égaux (environ 1,41), si bien que la composition, qui se présente au repos sous forme de deux phases limpides distinctes, séparées par une interface nette, reste complètement transparente après mélange des phases par agitation.

Après agitation, le mélange reste stable pendant plus de 30 secondes. Le déphasage s'effectue ensuite rapidement, et est complet en moins de deux minutes. L'interface entre les deux phases apparait alors à nouveau parfaitement nette.

Cette composition présente un effet amincissant particulièrement important, notamment en raison de la quantité élevée de caféine qu'elle contient, et de l'association entre la caféine et la phloridzine (présente dans la composition sous forme d'extrait de *Pirus malus*). Cet effet amincissant se traduit notamment par le traitement et la prévention des surcharges adipeuses et de la cellulite de la peau, par application de la composition par voie topique sur les zones de la peau concernées.

La composition reste en outre avantageusement stable pendant au moins 2 mois conservée à température ambiante, c'est-à-dire entre 20 et 25 °C environ, mais aussi en conditions de stress, plus particulièrement en condition de froid (à une température inférieure à 10 °C) et en condition de chaleur (à une température supérieure à 30 °C).

### EXEMPLE 2 - Soin anti-vieillissement cutané

Il est préparé une composition à effet anti-vieillissement cutané comprenant les ingrédients suivants. Pour chacun de ces ingrédients, la concentration est indiquée en pourcentage en poids, par rapport au poids total de la composition.

| Phase aqueuse | (30 %) |
|---|---|
| Glycérine | 10 - 20 % |
| Eau déminéralisée ou eau thermale | qsp 100 % |
| Extrait d'*Argania spinosa* | 0,0005 - 0,01 % |
| Acide citrique | 0 - 0,1 % |
| Maltodextrine | 0,05 - 0,2 % |
| Extrait de tige de *Pirus malus* | 0,05 - 0,3 % |
| Conservateur | 0 - 0,4 % |
| Colorant | qs |

| Phase huileuse | (70 %) |
|---|---|
| Diméthicone | 30 - 60 % |
| Coco caprylate | 10 - 25 % |
| Benzoate d'alkyle C12-15 | 0 - 5 % |
| Huile de noyau d'*Argania spinosa* | 0,1 - 5 % |
| Antioxydant | 0,01 - 0,1 % |
| Parfum | 0,05 - 0,5 % |

Les indices de réfraction de la phase aqueuse et de la phase huileuse sont avantageusement égaux (environ 1,41), si bien que la composition, qui se présente au repos sous forme de deux phases distinctes toutes deux limpides, séparées par une interface nette, est complètement transparente après mélange des phases par agitation.

Après agitation, le mélange reste stable pendant plus de 30 secondes. Le déphasage s'effectue ensuite rapidement, et est complet en moins de deux minutes. L'interface entre les deux phases apparait alors à nouveau parfaitement nette.

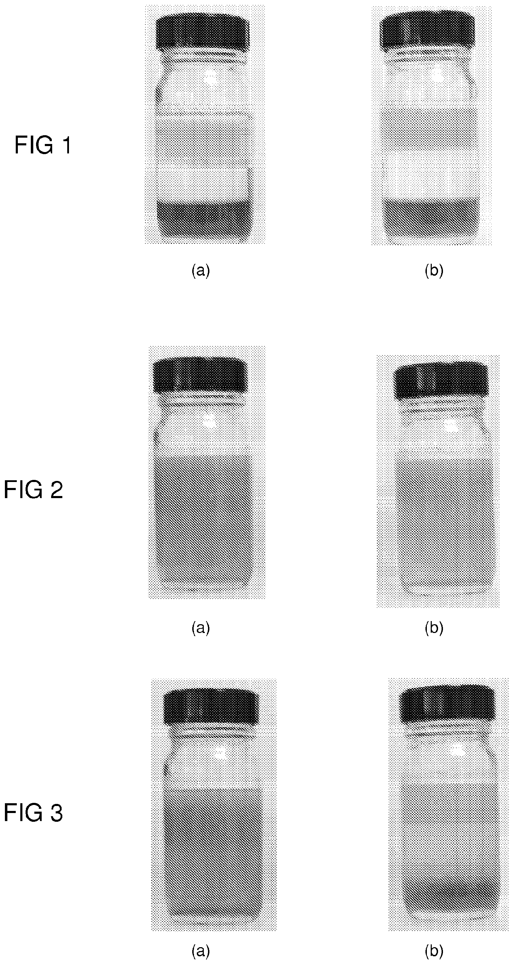

A titre comparatif, il a été préparé la même composition, mais sans maltodextrine. La figure 1 montre des flacons contenant, au repos, la composition selon l'invention (a) et la composition sans maltodextrine (b). On observe que, pour chacun des flacons, les deux phases sont bien distinctes, et l'interface entre les deux phases est nette.

Ces mêmes flacons sont montrés sur la figure 2, après agitation réalisée de sorte à mélanger la phase aqueuse et la phase huileuse. Le mélange y apparait homogène et transparent, pour les deux compositions testées.

Enfin, la figure 3 montre les deux mêmes flacons 11 secondes après l'arrêt de l'agitation. On observe clairement que pour la composition sans maltodextrine (b), un déphasage s'est déjà produit, la phase aqueuse colorée apparaissant nettement au fond du flacon. Au contraire, la composition selon l'invention, contenant de la maltodextrine (a), se trouve encore sous forme d'une dispersion homogène des deux phases. Cette dispersion reste stable durant un temps suffisant pour permettre à un utilisateur de prélever confortablement et sans se presser une dose homogène, pour utilisation.

Cette composition selon l'invention présente en outre un effet anti-âge important, notamment en raison de la présence simultanée de l'huile d'arganier, dans la phase huileuse, et de peptides issus de l'arganier, dans la phase aqueuse.

La composition reste en outre avantageusement stable pendant au moins 2 mois conservée à température ambiante, c'est-à-dire entre 20 et 25 °C environ, mais aussi en conditions de stress, plus particulièrement en condition de froid (à une température inférieure à 10 °C) et en condition de chaleur (à une température supérieure à 30 °C).

### EXEMPLE 3 - Composition cosmétique amincissante

Il est préparé une composition à effet amincissant comprenant les ingrédients suivants. Pour chacun de ces ingrédients, la concentration est indiquée en pourcentage en poids, par rapport au poids total de la composition.

| Phase aqueuse | (40 %) |
|---|---|
| Glycérine | 10 - 20 % |
| Eau déminéralisée ou eau thermale | qsp 100 % |
| Alcool | 1 - 15 % |
| Caféine | 1 % |
| Acide citrique | 0,005 % |
| Cyclodextrine | 0,05 - 0,2 % |
| Salicylate de triéthanolamine | 0,1 - 3,0 % |
| Chlorure de sodium | 0,1 - 1,5 % |
| Colorant | qs |

| Phase huileuse | (60 %) |
|---|---|
| Diméthicone | 30 - 59 % |
| Dicaprylyl carbonate | 0 - 20 % |
| Huile de graine de *Camelina sativa* | 0,1 - 4 % |
| Diméthiconol | 0 - 0,1 % |
| Parfum | 0,1 - 1 % |

Les indices de réfraction de la phase aqueuse et de la phase huileuse sont avantageusement égaux, si bien que la composition, qui se présente au repos sous forme de deux phases limpides distinctes, superposées l'une sur l'autre et séparées par une interface plane nette, reste complètement transparente après mélange des phases par agitation.

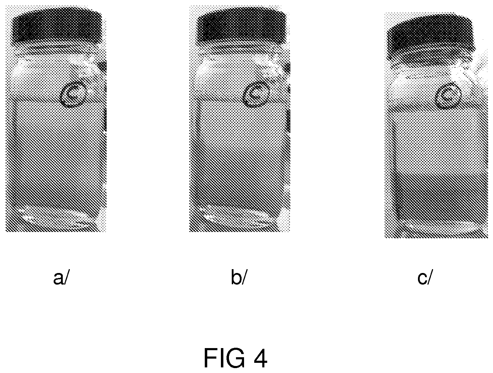

Après agitation, le mélange reste stable pendant plus de 30 secondes. Le déphasage s'effectue ensuite rapidement. La figure 4 montre des photographies de la composition à différents temps après la fin de l'agitation. Comme on peut le voir sur cette figure, après 50 secondes le déphasage a débuté. Il est complet en moins de deux minutes et quinze secondes (en c/ sur la figure).

Cette composition présente un effet amincissant particulièrement important. Elle reste en outre avantageusement stable pendant au moins 2 mois conservée à température ambiante, mais aussi en conditions de stress.

## Revendications

1. Composition cosmétique et/ou dermatologique, comprenant une phase aqueuse et une phase huileuse distinctes l'une de l'autre et superposées l'une sur l'autre, **caractérisée en ce que** la phase aqueuse contient une quantité comprise entre 0,05 et 0,2 % en poids, par rapport au poids total de la composition, d'un agent d'encapsulation de la phase huileuse choisi parmi les dextrines.

2. Composition selon la revendication 1, dans laquelle la dextrine est choisie parmi les maltodextrines et les cyclodextrines, et leurs mélanges.

3. Composition selon l'une quelconque des revendications 1 à 2, sensiblement exempte d'agent émulsionnant.

4. Composition selon l'une quelconque des revendications 1 à 3, dans laquelle les indices de réfraction de la phase aqueuse et de la phase huileuse sont sensiblement égaux.

5. Composition selon l'une quelconque des revendications 1 à 4, dans laquelle la phase huileuse contient une huile de silicone, ou un mélange de telles huiles.

6. Composition selon la revendication 5, dans laquelle la quantité totale d'huiles de silicone est comprise entre 30 et 60 % en poids par rapport au poids total de la composition.

7. Composition selon l'une quelconque des revendications 1 à 6, dans laquelle la phase aqueuse contient un polyol ou un mélange de polyols.

8. Composition selon la revendication 7, dans laquelle la quantité totale de polyols est comprise entre 10 et 20 % en poids par rapport au poids total de la composition.

9. Composition selon l'une quelconque des revendications 1 à 8, dans laquelle la phase aqueuse contient un alcool en C1-C6, dans une quantité inférieure à 15 % en poids par rapport au poids total de la composition.

10. Composition selon l'une quelconque des revendications 1 à 9, dans laquelle la phase huileuse représente 50 à 80 % en poids du poids total de la composition.

11. Composition selon l'une quelconque des revendications 1 à 10, contenant au moins un agent actif par voie topique

12. Composition selon la revendication 11, contenant au moins un agent actif à propriétés amincissantes.

13. Composition selon la revendication 12, contenant en tant qu'agent actif à propriétés amincissantes, de la caféine.

14. Composition selon l'une quelconque des revendications 12 à 13, contenant en tant qu'agent actif à propriétés amincissantes de la phloridzine.

15. Composition selon la revendication 11, contenant au moins un agent anti-vieillissement cutané.

16. Composition selon la revendication 15, dans laquelle la phase huileuse contient de l'huile d'*Argania Spinosa* et/ou la phase aqueuse contient un extrait hydrophile d'*Argania Spinosa.*

17. Utilisation non-thérapeutique d'une composition selon l'une quelconque des revendications 1 à 16 pour le soin cosmétique par application par voie topique sur la peau, les muqueuses et/ou les cheveux.

18. Utilisation selon la revendication 17, comprenant l'agitation de la composition de sorte à disperser la phase aqueuse et la phase huileuse l'une dans l'autre, et le prélèvement d'une dose de la dispersion ainsi formée pour application.

19. Utilisation non-thérapeutique selon l'une quelconque des revendications 17 à 18, pour le traitement et/ou la prévention des surcharges adipeuses localisées et/ou de la cellulite et/ou pour l'obtention d'un effet amincissant, la composition contenant au moins un agent actif à propriétés amincissantes.

20. Utilisation non-thérapeutique selon l'une quelconque des revendications 17 à 18, pour lutter contre le vieillissement cutané, la composition contenant au moins un agent anti-vieillissement cutané.

## Patentansprüche

1. Kosmetische und/oder dermatologische Zusammensetzung, die eine wässrige Phase und eine ölige Phase umfasst, welche voneinander getrennt sind und übereinanderliegen, **dadurch gekennzeichnet, dass** die wässrige Phase auf das Gesamtgewicht der Zusammensetzung bezogen eine Menge zwischen 0,05 und 0,2 Gewichts-% eines Mittels zum Einkapseln der öligen Phase enthält, das aus Dextrinen ausgewählt ist.

2. Zusammensetzung nach Anspruch 1, wobei das Dextrin aus Maltodextrinen und Cyclodextrinen und deren Mischungen ausgewählt ist.

3. Zusammensetzung nach einem der Ansprüche 1 bis 2, die im Wesentlichen frei von Emulsionsmittel ist.

4. Zusammensetzung nach einem der Ansprüche 1 bis 3, wobei die Brechungsindizes der wässrigen Phase und der öligen Phase im Wesentlichen gleich sind.

5. Zusammensetzung nach einem der Ansprüche 1 bis 4, wobei die ölige Phase ein Silikonöl oder eine Mischung solcher Öle enthält.

6. Zusammensetzung nach Anspruch 5, wobei die Gesamtmenge an Silikonölen auf das Gesamtgewicht der Zusammensetzung bezogen zwischen 30 und 60 Gewichts-% beträgt.

7. Zusammensetzung nach einem der Ansprüche 1 bis 6, wobei die wässrige Phase ein Polyol oder eine Mischung von Polyolen enthält.

8. Zusammensetzung nach Anspruch 7, wobei die Gesamtmenge an Polyolen auf das Gesamtgewicht der Zusammensetzung bezogen zwischen 10 und 20 Gewichts-% beträgt.

9. Zusammensetzung nach einem der Ansprüche 1 bis 8, wobei die wässrige Phase einen C1-C6-Alkohol in einer Menge, auf das Gesamtgewicht der Zusammensetzung bezogen, von weniger als 15 Gewichts-% enthält.

10. Zusammensetzung nach einem der Ansprüche 1 bis 9, wobei die ölige Phase 50 bis 80 Gewichts-% des Gesamtgewichts der Zusammensetzung ausmacht.

11. Zusammensetzung nach einem der Ansprüche 1 bis 10, die mindestens einen topischen Wirkstoff enthält.

12. Zusammensetzung nach Anspruch 11, die mindestens einen Wirkstoff mit schlankmachenden Eigenschaften enthält.

13. Zusammensetzung nach Anspruch 12, die als Wirkstoff mit schlankmachenden Eigenschaften Koffein enthält.

14. Zusammensetzung nach einem der Ansprüche 12 bis 13, die als Wirkstoff mit schlankmachenden Eigenschaften Phloridzin enthält.

15. Zusammensetzung nach Anspruch 11, die mindestens ein Mittel gegen Hautalterung enthält.

16. Zusammensetzung nach Anspruch 15, wobei die ölige Phase *Argania Spinosa-*Öl enthält und/oder die wässrige Phase einen hydrophilen *Argania Spinosa*-Extrakt enthält.

17. Nicht-therapeutische Verwendung einer Zusammensetzung nach einem der Ansprüche 1 bis 16 zur kosmetischen Pflege durch topische Anwendung auf der Haut, den Schleimhäuten und/oder den Haaren.

18. Verwendung nach Anspruch 17, die das Schütteln der Zusammensetzung, um die wässrige Phase und die ölige Phase ineinander zu dispergieren, und das Entnehmen einer Dosis der so gebildeten Dispersion zur Anwendung umfasst.

19. Nicht-therapeutische Verwendung nach einem der Ansprüche 17 bis 18 zur Behandlung und/oder Vorbeugung von lokalisierten Fettdepots und/oder Cellulite und/oder zum Erhalten eines schlankmachenden Effekts, wobei die Zusammensetzung mindestens einen Wirkstoff mit schlankmachenden Eigenschaften enthält.

20. Nicht-therapeutische Verwendung nach einem der Ansprüche 17 bis 18, um die Hautalterung zu bekämpfen, wobei die Zusammensetzung mindestens ein Mittel gegen Hautalterung enthält.

## Claims

1. Cosmetic and/or dermatological composition, comprising an aqueous phase and an oily phase which are distinct from each other and are superimposed on each other, **characterized in that** the aqueous phase contains an amount of between 0.05 and 0.2% by weight, with respect to the total weight of the composition, of an encapsulating agent for encapsulating the oily phase, chosen from dextrins.

2. Composition according to Claim 1, wherein the encapsulating agent is chosen from maltodextrins and cyclodextrins, and their mixtures.

3. Composition according to any one of Claims 1 to 2, substantially devoid of emulsifying agent.

4. Composition according to any one of Claims 1 to 3, wherein the refractive indices of the aqueous phase and of the oily phase are substantially equal.

5. Composition according to any one of Claims 1 to 4, wherein the oily phase contains a silicone oil, or a mixture of such oils.

6. Composition according to Claim 5, wherein the total amount of silicone oils is between 30 and 60% by weight, with respect to the total weight of the composition.

7. Composition according to any one of Claims 1 to 6, wherein the aqueous phase contains a polyol or a mixture of polyols.

8. Composition according to Claim 7, wherein the total amount of polyols is between 10 and 20% by weight, with respect to the total weight of the composition.

9. Composition according to any one of Claims 1 to 8, wherein the aqueous phase contains a C₁-C₆ alcohol, in an amount of less than 15% by weight, with respect to the total weight of the composition.

10. Composition according to any one of Claims 1 to 9, wherein the oily phase represents from 50 to 80% by weight, of the total weight of the composition.

11. Composition according to any one of Claims 1 to 10, containing at least one agent which is active by the topical route.

12. Composition according to Claim 11, containing at least one active agent having slimming properties.

13. Composition according to Claim 12, containing caffeine as active agent having slimming properties.

14. Composition according to any of claims 12 to 13, comprising phloridzin as active agent having slimming properties.

15. Composition according to Claim 11, containing at least one skin anti-ageing agent.

16. Composition according to Claim 15, wherein the oily phase contains *Argania spinosa* oil and/or the aqueous phase contains a hydrophilic *Argania spinosa* extract.

17. Non-therapeutic use of a composition according to any one of Claims 1 to 16 for cosmetic care by application by the topical route to the skin, the mucous membranes and/or the hair.

18. Use according to Claim 17, comprising the stirring of the composition, so as to disperse the aqueous phase and the oily phase in each other, and the withdrawal of a quantity of the dispersion thus formed for application.

19. Non-therapeutic use according to either one of Claims 17 and 18 for the treatment and/or the prevention of localized excess fat deposits and/or of cellulite and/or for obtaining a slimming effect, the composition containing at least one active agent having slimming properties.

20. Non-therapeutic use according to either one of Claims 17 and 18 for combating skin ageing, the composition containing at least one skin anti-ageing agent.
